# EUROPEAN PATENT APPLICATION

(11) **EP 2 660 234 A1**
(43) Date of publication of application: **06.11.2013**
(21) Application number: 12305491.8
(22) Date of filing: 02.05.2012
(51) Int. Cl.: C07C 51/36, C07C 57/26

(54) **Process for the production of artemisinin intermediates**

(71) Applicant: SANOFI, 75008 Paris (FR)
(72) Inventor: Burgard, Andreas, 65926 Frankfurt am Main (DE); Feth, Martin, Philipp, 65926 Frankfurt am Main (DE); Rossen, Kai, 65926 Frankfurt am Main (DE)
(74) Representative: Domenego, Bertrand

(57) **Abstract**

This application relates to a process for the production of (2R)-dihydroartemisinic acid or (2R)-dihydroartemisinic acid esters from artemisinic acid or artemisinic acid esters, respectively, by diimine hydrogenation of the exocyclic CC-double bond under an air comprising low oxygen concentration, and use of said process in the production of the antimalarial drug artemisinin.

## Description

Malaria is a tropical disease and is common in Africa, South East Asia, and South America. Approximately 300-500 million people are infected with malaria, making it one of the world's major infectious diseases. In 2006, an estimated 1.5 to 2.7 million deaths resulted from malaria and most of the deaths occurred in children under five years old. Disease control is hampered by the occurrence of multi-drug resistant strains of the parasite *Plasmodium falciparum.* Therefore it is an important world health objective to develop new anti-malaria drugs, and alternative methods of producing anti-malaria drugs. One of these anti-malaria drugs is artemisinin of the formula II, 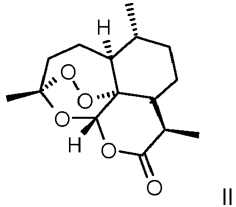 a sesquiterpene lactone endoperoxide which is a component of the traditional Chinese medical herb *Artemisia annua.* It has been utilized for controlling symptoms of fever in China for over 1000 years. In the scientific literature, artemisinin is also sometimes referred to by its Chinese name Qinghaosu. Recent strides have been made in understanding the properties and structure of this molecule. The compound was first isolated in 1972, and its anti-malaria activity was discovered in 1979. The first total synthesis of the molecule was accomplished by chemists at Hoffmann-La Roche in 1983 (G. Schmidt, W. Hofheinz, J. Am. Chem. Soc., 105, 624 (1983)). Artemisinin is highly effective against multi-drug resistant *Plasmodium spp.,* but is in short supply and unaffordable to most malaria sufferers. The production of artemisinin can be accomplished through several routes. For instance, methods involving extracting artemisinin from *Artemisia annua* (Wallaart et al., Planta Med; 66, 57 (2000); Abdin et al., Planta Med; 69, 289 (2003)), or methods involving extracting the biosynthetic precursor molecule artemisinic acid of formula IIIa, 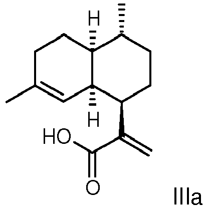 from *Artemisia annua* and then synthetically converting this molecule in several synthetic steps into artemisinin. However, these known methods fail to generate sufficient yields of artemisinin, and it is currently estimated that the world's supply of the plant would meet less than 10% of the world's demand for artemisinic acid and artemisinin (WO 2006/128126).

Another alternate production procedure is the total synthesis of artemisinin. However, such total synthesis involves a large number of synthetic steps and is not efficient and cost-effective for providing large amounts of the desired drug.

In 2006, scientists from Amyris Inc. and the University of California, Berkeley developed a fermentation process with engineered yeast to produce high titres of artemisinic acid of the formula IIIa. Specifically, the engineered yeast was used to produce artemisinic acid by using an engineered mevalonate pathway, amorphadiene synthase, and a novel cytochrome P450 monooxygenase from *Artemisia annua* that performs a three step oxidation of amorpha-4,11-diene to the intermediate of formula IIIa (J.D. Keasling et al., Nature, 440, 940 (2006)). Two years later, the titres were increased to even higher and even more economical levels (R. Regentin et al., Biotechnol. Prog.; 24, 1026 (2008)). Since these new developments, semi-synthesis methods involving the use of biosynthetic precursor like artemisinic acid of the formula IIIa (produced by fermentation of a genetically engineered microorganism) are considered to be cost-effective, environmentally friendly, high quality, and reliable source of artemisinin.

One of the critical steps during the production of artemisinin is the regio- and stereoselective reduction of artemisinic acid (formula IIIa) to diastereomeric dihydroartemisinic acid (formula la).

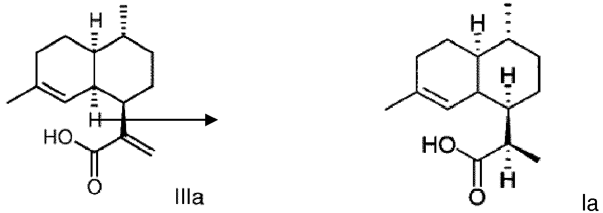

Such reduction step can be carried out by hydrogenation under pressure in the presence of chiral transition metal catalysts, which are relatively expensive and need to be removed after the reaction.

As an alternative to the chiral hydrogenation of artemisinic acid, WO 2011/030223 discloses a process in which artemisinic acid is reduced into dihydroartemisinic acid in the presence of diimide (diimine, diazene). WO 2011/030223 as well as Org. Reactions 40, 91-155, 1991 by D.J. Pasto and R.T. Taylor describe various methods for generating diimide reducing agent.

Specifically, WO2011/030223 describes the *in situ* formation of diimide by an oxidation of hydrazine or hydrazine hydrate with oxidants (such as hydrogen peroxide (H₂O₂)) and oxygen with and without metal catalysts (for example copper salts) or organic catalysts (for example flavine derivatives). When oxygen is used as an oxidant for the diimide formation from hydrazine, only the use of air (contains 21% by volume of O₂) or pure oxygen has been described.

However, the use of flammable solvents such as alcohols in chemical reactions in the presence of air having 21% by volume of O₂ is potentially very dangerous, let alone the use of pure oxygen. For alcohols such as ethanol and isopropanol, the oxygen limit concentration at room temperature and atmospheric pressure is less than 9% by volume. Above the oxygen limit concentration, safe handling in combination with flammable solvents is no longer ensured. Accordingly, on an industrial scale, synthetic air above the oxygen limit concentration is often prohibited.

In addition, in industrial setting, air with a low oxygen concentration is often used to ensure that oxidative processes would not take place. That is, low content of oxygen is selected to avoid or minimize chemical oxidation reaction(s).

The inventors of the present disclosure surprisingly discovered that the reduction of artemisinic acid to dihydroartemisinic acid in the presence of hydrazine and/or hydrazine hydrate can be completed under a low oxygen condition.

Thus, provided herein is a process for the preparation of a compound of formula I, 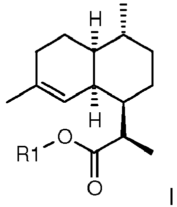 wherein R1 is hydrogen or linear or branched alkyl having 1, 2, 3, 4, 5, or 6 carbon atoms or cycloalkyl having 3, 4,5, 6, or 7 carbon atoms, starting either from commercially available materials or compounds already described in the literature, themselves being prepared easily from commercially available materials. Simple and environmentally compatible reagents and solvents may be used to afford high overall yields and good purity of the products and that can be performed on an industrial scale.

The compound of the formula I may be obtained by reacting a compound of formula III 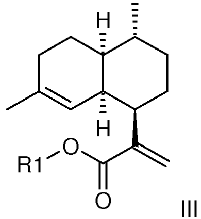 with hydrazine and/or hydrazine hydrate under an air comprising a low concentration of oxygen. In some embodiments, diimine is generated *in situ* during the reaction. Also in some embodiments, the air having a low concentration of oxygen may comprise oxygen in an amount less than or equal to 20%, such as less than or equal to 15%, also such as less than or equal to 10%, further such as less than or equal to 8%, and even further such as less than or equal to 5% by volume relative to the total volume of air. The process may result in high yield and good regio- and diastereoselectivity.

Also provided is a process for preparing a compound of the formula I: 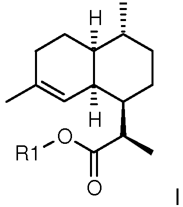 wherein
R1 is hydrogen or linear or branched alkyl having 1, 2, 3, 4, 5 or 6 carbon atoms or cycloalkyl having 3, 4, 5, 6 or 7 carbon atoms,
comprising reacting a compound of the formula III 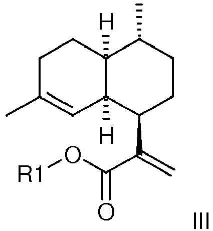
with hydrazine and/or hydrazine hydrate under an air comprising a low concentration of oxygen.

In some embodiments, R1 is hydrogen.

Diimine is a reactive reagent which is also called diimide or diazene and has the chemical formula HN=NH.

Hydrazine, also called diamine or diazane, has the chemical formula N₂H₄.

Hydrazine hydrate, also called hydrazinium hydroxide, has the chemical formula N₂H₄·H₂O.

In some embodiments, the process of reacting a compound of the formula III with hydrazine and/or hydrazine hydrate is conducted under or with an air comprising a low concentration of oxygen, such as an air comprising oxygen at a concentration of less than or equal to 20%, less than or equal to 15%, less than or equal to 10%, less than or equal to 8 %, or less than or equal to 5% by volume relative to the total volume of the air. As a non-limiting example, the process of reacting a compound of the formula III with hydrazine and/or hydrazine hydrate is conducted under or with an air comprising 5% by volume relative to the total volume of the air of oxygen and 95% by volume relative to the total volume of the air of nitrogen (i.e, 5% O₂ + 95% N₂). As another non-limiting example, the process of reacting a compound of the formula III with hydrazine and/or hydrazine hydrate is conducted under or with an air comprising oxygen less than or equal to oxygen limit concentration. "Oxygen limit concentration," also known as "limiting oxygen concentration" or "minimum oxygen concentration," is the maximum oxygen concentration in a mixture of an inflammable material with air which does not explode in combination with arbitrary fuel concentrations. Oxygen limit concentration is dependant on pressure, temperature, the type of air (e.g., the type of inert gas present in the air) and may be determined by methods known to those skilled in the art.

In some embodiments, the process of reacting a compound of the formula III is conducted with at least one solvent. As a non-limiting example, the at least one solvent comprises at least one alcohol. Further as a non-limiting example, the at least one solvent comprises at least one alcohol and water. In some embodiments, the at least one alcohol is chosen from methanol, ethanol, isopropanol, and trifluoroethanol. Even further as a non-limiting example, the at least one solvent is chosen such that it has a solubility suitable for oxygen, artemisinic acid, and/or dihydroartemisinic acid.

In certain embodiments, the at least one solvent is chosen from ethanol and isopropanol.

The choice of solvent may have an effect on the reaction rate of the compound of formula III. For instance, the inventors have surprisingly found out that ethanol and isopropanol may provide a higher conversion rate of the formula III to formula I than trifluoroethanol. As non-limiting examples, the reduction of artemisinic acid to dihydroartemisinic acid at 40-50°C in the presence of 2-3 equivalents of hydrazine hydrate may be completed in ethanol in 17-23 h and in isopropanol in 8-10 h, the reaction solution being gassed with 5% strength synthetic air (5% by volume of oxygen + 95% by volume of nitrogen). The addition of gas was carried out by continuously pumping 5% strength synthetic air through the reaction solution through a glass frit or a gas-permeable bottom plate which allows distribution of small gas bubbles. By comparison, when trifluoroethanol was used as a solvent, the conversion of artemisinic acid into dihydroartemisinic acid after 7.5 h of gassing with 5% strength synthetic air in the presence of 3 equivalents of hydrazine hydrate was 12.5%. The conversion was completed only after about 60 h. These results were rather surprising as trifluoroethanol is known to be a better solvent for oxygen than ethanol and isopropanol (the solubility of oxygen in molar fractions of 10⁴X₁ oxygen dissolved in the solvent in question at 283.15 K and 1013 mbar is stated as 5.96 for ethanol, 8.00 for isopropanol and 9.29 for trifluoroethanol (R. Battino et al., J. Phys.Chem. Ref. Data, Vol. 12, 2, 1983, 163-178 and M.A. Sanchez et al., Can. J. Chem. 79, 2001, 1460-1465)), and it would have been assumed that the conversion of artemisinic acid into dihydroartemisinic acid would proceed better in trifluoroethanol than in ethanol or isopropanol.

In some embodiments, the reduction of artemisinic acid to dihydroartemisinic acid may be carried out in a solvent comprising at least one alcohol and water. As a non-limiting example, the solvent may comprise water and at least one alcohol chosen from methanol, ethanol, and isopropanol, wherein the water is present in an amount less than or equal to 50% by volume relative to the total volume of the solvent. In some embodiments, the amount of water is selected to obtain an appropriate conversion rate. The diastereoselective reduction of artemisinic acid (formula la) to dihydroartemisinic acid (formula IIIa) with hydrazine hydrate in water only did not proceed well, even in an atmosphere of pure O₂. Thus, the at least one alcohol may play an important role in the diastereoselective reduction of artemisinic acid (formula la) to dihydroartemisinic acid (formula IIIa).

In some embodiments, the reduction of artemisinic acid (formula la) to dihydroartemisinic acid (formula IIIa) is conducted at a temperature ranging from 20 °C to 80 °C, such as at a temperature ranging from 30 °C to 70 °C, further such as at a temperature ranging from 35 °C to 60 °C, even further such as at a temperature ranging from 40 °C to 50 °C.

In some other embodiments, the reduction of artemisinic acid (formula la) to dihydroartemisinic acid (formula IIIa) may be conducted at a temperature below 40 °C such as at a room temperature of 25 °C; however, this may require a longer reaction time for the completion of the reaction.

The reaction rates are dependent on the reaction temperature, the solvents, and the mixing conditions applied to the reactive components in the reaction medium. In some embodiments, micro-mixing techniques may be useful to achieve advantageous turnover rates of substrates.

The reaction times are variable and depend on the reaction scale, the base, the solvent, and the temperature selected for this process. In some embodiments, reaction times may range from 0.5 hours to 24 hours.

In some embodiments, the processes disclosed herein for converting formula III to formula I may be carried out without the use of any additional catalysts such as noble metal catalysts or 5-ethyl-3-methyllumiflavinium perchlorate. In other words, the processes disclosed herein do not use any additional catalyst system for converting formula III to formula I. The use of additional catalysts may lead to a higher manufacturing cost (e.g., some noble metal catalysts are relative expansive and need to be removed with additional separation step after the reaction). Thus, processes that can be implemented without any additional catalysts may be more cost-effective than the ones that require the use of additional catalysts.

In some embodiments, the processes disclosed herein lead to a mixture comprising the formula I and formula IV, wherein the ratio of the formula I and formula IV is better than 90% to 10%, for instance, the ratio of the formula I and formula IV is at least 95% to 5%, at least 97% to 3%, or at leat 99% to 1%.

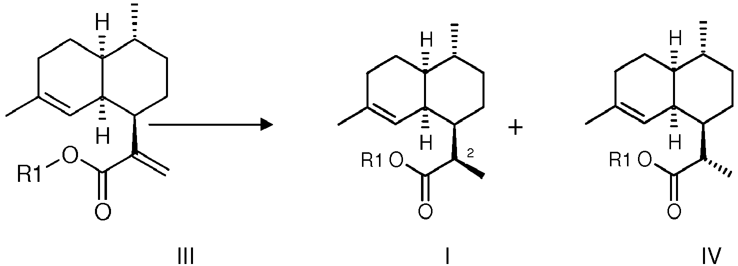

The reaction turnover may be controlled by monitoring the reaction, for example using reversed phase high pressure liquid chromatography techniques (RP-HPLC), before submitting the mixture to the work-up procedure. After complete consumption of the substrate, the compound of the formula I obtained by the process can be isolated by methods known to those skilled in the art. These procedures can include an aqueous work-up of the reaction mixture or a chromatography of the reaction mixture. An example of a convenient work-up procedure involves acidifying the reaction mixture, for instance with hydrochloric acid, and then extraction of the desired product of the formula I, for example by a standard extraction with a water immiscible solvent like 2-methyl-tetrahydrofuran, dichloromethane, methyl-tert-butylether (MTBE), toluene, ethyl acetate, heptane, cyclohexane, methyl-isobutylketone, benzene or isobutyl acetate. In some embodiments, the water immiscible solvent is dichloromethane or MTBE. Standard aqueous work-up procedures allow for the isolation of the compound of formula I. Alternatively, the desired product can be obtained by a chromatographic purification or by crystallization. The diastereomeric ratio of the crude products of formulae I:IV that can be achieved by this procedure without crystallization is typically better than 90%:10% and can approach a ratio of 99%:1%. In addition, the diastereomeric purity of the product can be enhanced to 100% by crystallization.

In some embodiments, the process for preparing the compound of the formula I: 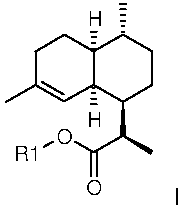 comprising esterifying a compound of the formula IIIa 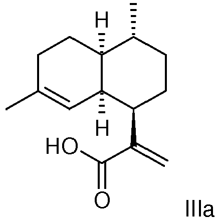 to produce a compound of the formula III 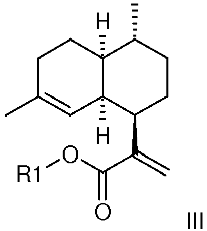 and the compound of the formula III is subsequently reacted with hydrazine and/or hydrazine hydrate under an air comprising a low concentration of oxygen as described above.

Sufficient quantities of artemisinic acid IIIa may be available from several sources, e.g., from plant extraction of Artemisia annua (R.J. Roth et al., Planta Med; 53, 501 (1987)), or from the yeast fermentation process as described by J. D. Keasling et al. (Nature, 440, 940 (2006)).

Esterification of the compound of the formula IIIa can be performed by a method known in the art, e.g. as described by W. Greene et al.; Protective Groups in Organic Synthesis; Wiley-Interscience 3rd ed., chapter 5 (1999). For example, esterification reactions can be performed by the following methods:
(i) by reacting compound IIIa with a compound R1-X, wherein X is F, Cl, Br, or I, and in some embodiments, X is Cl or Br; or
(ii) by reacting compound IIIa with a diazoalkane (e.g., with diazomethane to provide the methylester); or
(iii) by preparing an acid chloride of the formula IIIb from compound IIIa 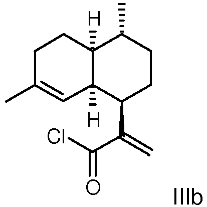 by methods known in the art, and subsequently reacting the acid chloride with an alcohol of formula R1-OH; or
(iv) by condensation of a compound of the formula IIIa with the respective alcohol of formula R1-OH in the presence of carbonyldiimidazole, a carbodiimide or a chloroformate.

As non-limiting examples, X. Xu et al., Tetrahedron 42, 819 (1986), describes the synthesis of artemisinic acid methyl ester (a compound of the formula III wherein R1 is methyl) by reacting artemisinic acid with CH₂N₂, for example, in ether at 0 °C. Zhou et al. (Huaxue Xuebao 43(9), 845-851, 1985) describes the synthesis of artemisinic acid tert-butyl ester by generating a mixed anhydride of artemisinic acid with pivaloylchloride and treating the product with sodium tert-butanolate.

The compound of the formula I can be oxidized to arteminisin of the formula II by methods known in the art, e.g. as described by X. Xu et al. (Tetrahedron; 42, 819 (1986)), R.J. Roth et al. (U.S. 4,992,561) and K. Reiling et al. (WO 2006/128126), i.e. by oxygenation of a compound of the formula I with hydrogen peroxide and sodium molybdate dihydrate, followed by a second oxidation with oxygen in the presence of copper (II) trifluoromethanesulfonate to yield arteminisin of the formula II.

In the compound of the formula I wherein R1 is linear or branched alkyl having 1, 2, 3, 4, 5 or 6 carbon atoms or cycloalkyl having 3, 4, 5, 6 or 7 carbon atoms, the ester compound may be previously cleaved to a compound of the formula I wherein R1 is H by methods known in the art as described by W. Greene et al., Protective Groups in Organic Synthesis, Wiley-Interscience 3rd ed., e.g. by reacting the said compound with a base.

### EXAMPLES

The following examples are provided by way of illustration only and not by way of limitation. Those of skill in the art will readily recognize a variety of parameters that could be changed or modified to yield similar results.

### Example 1: Reduction of artemisinic acid to dihydroartemisinic acid in ethanol

15 g (64 mmol) of artemisinic acid were initially charged in 45 ml of ethanol. At 15-25 °C, 9.81 g (192 mmol) of hydrazine hydrate were added dropwise to the artemisinic acid solution. The reaction solution was then transferred into a heatable chromatography column with bottom glass frit and, at 40 °C, gassed with 5% strength synthetic air (5% O₂ + 95% N₂) via the bottom glass frit for 22 hours. After complete conversion into dihydroartemisinic acid (checked by Raman spectroscopy) the reaction solution was discharged into a vessel and the chromatography column was washed twice with 15 ml of ethanol. The reaction solution (pH 8) was adjusted to pH 5 using about 40 ml of 2N hydrochloric acid and diluted with 110 ml of water. After 20 minutes of stirring, the crystal suspension was filtered off and the filter cake was washed with 15 ml of water. The moist crystalline dihydroartemisinic acid was dried under reduced pressure at 40 °C overnight. This gave 13.9 g of dihydroartemisinic acid in a yield of 91.8% and a diastereomer ratio of 97:3 (HPLC) as a white crystalline solid.

### Example 2: Reduction of artemisinic acid to dihydroartemisinic acid in isopropanol

### a) Dihydroartemisinic acid, crystalline

15 g (64 mmol) of artemisinic acid were initially charged in 45 ml of isopropanol. At 15-25 °C, 9.81 g (192 mmol) of hydrazine hydrate were added dropwise to the artemisinic acid solution. The reaction solution was then transferred into a heatable chromatography column with bottom glass frit and, at 40 °C, gassed with 5% strength synthetic air (5% O₂ + 95% N₂) via the bottom glass frit for 10 hours. After complete conversion into dihydroartemisinic acid (checked by Raman spectroscopy) the reaction solution was discharged into a vessel and the chromatography column was washed twice with 15 ml of isopropanol. The reaction solution (pH 8) was adjusted to pH 5 using about 40 ml of 2N hydrochloric acid and diluted with 110 ml of water. After 20 minutes of stirring, the crystal suspension was filtered off and the filter cake was washed with 15 ml of water. The moist crystalline dihydroartemisinic acid was dried under reduced pressure at 40 °C overnight. This gave 13.7 g of dihydroartemisinic acid in a yield of 90.5% and a diastereomer ratio of 97:3 (HPLC) as a white crystalline solid.

### b) Dihydroartemisinic acid, dissolved in methylene chloride

15 g (64 mmol) of artemisinic acid were initially charged in 45 ml of isopropanol. At 15-25 °C, 9.81 g (192 mmol) of hydrazine hydrate were added dropwise to the artemisinic acid solution. The reaction solution were then transferred into a heatable chromatography column with bottom glass frit and, at 40 °C, gassed with 5% strength synthetic air (5% O₂ + 95% N₂) via the bottom glass frit for 10 hours. After complete conversion into dihydroartemisinic acid (checked by Raman spectroscopy) the reaction solution was discharged into a vessel and the chromatography column was washed twice with 15 ml of isopropanol. The reaction solution (pH 8) was adjusted to pH 3-4 using at least 40 ml of 2N hydrochloric acid. After addition of 75 ml of methylene chloride, the two-phase mixture was stirred vigorously for 10 minutes. Organic and aqueous phase were then separated. The organic phase was extracted once more with 50 ml of water. The resulting methylene chloride solution comprises 14.2 g of dihydroartemisinic acid in a yield of 94% and a diastereomer ratio of 97:3 (HPLC).

### Example 3: Reduction of artemisinic acid to dihydroartemisinic acid in trifluoroethanol

The following data were obtained according to the procedure as given in Example 1, except that 20% oxygen (synthetic air) was used. The data show that the use of trifluoroethanol, even though this solvent allows higher concentrations of oxygen in the liquid phase, results in lower reactivity in the reaction of artemisinic acid (AA) in the presence of 2 equivalents hydrazinhydrate dihydroartemisinic acid (DHAA) compared to ethanol or isopropanol.

| At room temperature RT | | | | |
|---|---|---|---|---|
| Solvent | AA | | DHAA | Time |
| Ethanol | 50 | | 50 | after 18 h |
| Isopropanol | 40 | | 60 | after 21 h |
| CF₃CH₂OH | 60 | | 40 | after 60 h |
| Ethanol/H₂O (1:1 volume ratio) | 70 | | 30 | after 19 h |

| At 40 °C | | | | |
|---|---|---|---|---|
| Solvent | AA | | DHAA | Time |
| CF₃CH₂OH | 65 | | 35 | after 7 h |
| Ethanol/H₂O (2: 1 volume ratio) | 65 | | 35 | after 2 h |
| Ethanol | 10 | | 90 | after 21 h |
| Isopropanol | 0 | | 100 | after 10 h |

## Claims

1. A process for the preparation of a compound of formula I 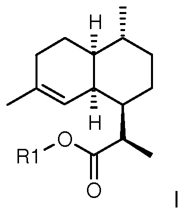 wherein R1 is hydrogen or linear or branched alkyl having 1, 2, 3, 4, 5 or 6 carbon atoms or cycloalkyl having 3, 4, 5, 6 or 7 carbon atoms,
comprising reacting a compound of formula III 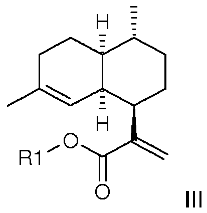 with hydrazine and/or hydrazine hydrate under an air comprising a low concentration of oxygen.

2. The process according to claim 1, wherein diimine is generated *in situ* during the reaction.

3. The process according to claim 1 or 2, wherein the air comprises oxygen in an amount less than or equal to 20% by volume relative to the total volume of the air.

4. The process according to any of the preceding claims, wherein the air comprises oxygen in an amount less than or equal to 10% by volume relative to the total volume of the air.

5. The process according to any of the preceding claims, wherein the air comprises oxygen in an amount less than or equal to 5% by volume relative to the total volume of the air and nitrogen in an amount more than or equal to 95% by volume relative to the total volume of the air.

6. The process according to any of the preceding claims, wherein the air comprises oxygen in an amount less than or equal to oxygen limit concentration.

7. The process according to any of the preceding claims, wherein the compound of the formula III is in a solvent comprising at least one alcohol.

8. The process according to claim 7, wherein the at least one alcohol is chosen from methanol, ethanol, and isopropanol.

9. The process according to claim 7 or 8, wherein the solvent further comprises water.

10. The process according to claim 9, wherein the water is present in an amount less than or equal to 50% by volume relative to the total volume of the solvent.

11. The process according to any of the preceding claims, wherein the reaction of the compound of the formula III is conducted at a temperature ranging from 20 °C to 80 °C.

12. The process according to any of the preceding claims, wherein the reaction of the compound of the formula III is conducted at a temperature ranging from 40 °C to 50 °C.

13. The process according to any of the preceding claims, further comprising oxidizing the compound of the formula I to artemisinin II
